# EUROPEAN PATENT APPLICATION

(11) **EP 4 327 806 A1**
(43) Date of publication of application: **28.02.2024**
(21) Application number: 22791078.3
(22) Date of filing: 20.04.2022
(51) Int. Cl.: A61K 9/48, A61K 9/54, A61P 1/00, A61P 1/16, A61P 13/12

(54) **CAPSULE FOR SPECIFIC DRUG DELIVERY AND PREPARATION METHOD THEREFOR**

(30) Priority: 20.04.2021 CN 202110423824
(71) Applicant: Jiangsu Hengrui Pharmaceuticals Co., Ltd., Lianyungang, Jiangsu 222047 (CN)
(72) Inventor: SUN, Haifeng, Lianyungang, Jiangsu 222047 (CN); WANG, Weibi, Lianyungang, Jiangsu 222047 (CN); WANG, Wei, Lianyungang, Jiangsu 222047 (CN); WANG, Jie, Lianyungang, Jiangsu 222047 (CN); CAO, Xiaoli, Lianyungang, Jiangsu 222047 (CN); MO, Zhirong, Lianyungang, Jiangsu 222047 (CN)
(74) Representative: dompatent von Kreisler Selting Werner - Partnerschaft von Patent- und Rechtsanwälten mbB
(86) International application number: PCT/CN2022/087994
(87) International publication number: WO 2022/222972

(57) **Abstract**

A capsule for specific drug delivery and a preparation method therefor, relating to a capsule containing a non-liquid filler and a modified release coating. The modified release coating covers a capsule shell, an isolating layer is provided between the capsule shell and the modified release coating, and no sealing film is provided between the body and cap of the capsule. The capsule can be used for delivering a drug to an intestinal track, and exhibits excellent characteristics in mass production of capsules and drug release.

## Description

The present application claims priority to Chinese Patent Application No. 202110423824X filed on April 20, 2021, which is incorporated herein by reference in its entirety.

### TECHNICAL FIELD

The present disclosure belongs to the field of pharmaceutical formulations, and particularly relates to a capsule for specific drug delivery and a preparation method therefor.

### BACKGROUND

Patients have good compliance with the administration of oral formulations due to convenience. Oral formulations typically pass through the stomach into the intestinal tract. However, protein-or polypeptide-based drugs can be easily denatured and degraded by gastric acid and digestive enzymes in the stomach. As a result, administering them orally becomes challenging. Furthermore, the treatment of diseases such as Crohn's disease and ulcerative colitis necessitates releasing drugs within the intestinal tract. However, oral administration leads to drug release and absorption in the stomach and the small intestine, making it difficult to achieve effective treatment at intended sites. Moreover, releases at non-target sites can readily lead to side effects. Hence, intestinal delivery systems have emerged in an effort to compensate for the limitations of conventional oral formulations. These systems target drug delivery at the intestinal tract, achieving targeted therapy and thereby diminishing toxic and side effects and reducing dosage requirements.

The smoothness or evenness of the surface of the shell of a common capsule such as a hydroxypropyl methylcellulose capsule also affects the delayed-release component coating in a later stage. In addition, there will be a gap or step when the body and cap of a capsule are joined. Capsules coated with the delayed-release component alone and filled cannot well meet the expectations due to the risk of leakage.

The prior art US6228396 discloses a colonic delivery starch capsule covered in an acrylic enteric coating. The capsule adopted by the prior art is an injection-molded starch capsule. The capsule has no step or gap between the body and the cap and thus can be easily coated. A disadvantage is that preparing such capsules needs special capsule filling machines and sealing machines.

The prior art CN106456554 discloses a capsule comprising a sustained-release coating. Before the capsule is coated, the gap between the body and the cap is sealed using a band-sealing technique. However, the technique requires special band-sealing equipment.

The prior art CN106265587 discloses an enteric-coated hollow capsule, which comprises a capsule shell and an enteric coating. The capsule shell is covered in the enteric coating, and the gap between the cap and body of the capsule is sealed with a sealing film. An isolating layer is arranged between the capsule shell and the enteric coating. The main ingredient of the isolating layer is hydroxypropyl methylcellulose. Simply adding a pre-coating layer between the delayed-release component and the capsule shell may solve the drug leakage problem or the delayed-release layer coating problem. However, in practical terms, capsule release will become a new problem. For this reason, there is still a need for a drug delivery composition for improving intestinal targeting. This composition has an improved capsule coating layer component which does not affect drug release from capsules and can eliminate steps and gaps between the body and cap of capsules, as well as cracks in the drying process after capsule coating. In addition, this composition is suitable for industrial production and its preparation needs no special equipment.

### SUMMARY

The present disclosure provides a capsule containing a non-liquid filler and comprising a modified-release coating, wherein the capsule shell is covered in the modified-release coating, and an isolating layer comprising a hydrophilic molecule is arranged between the capsule shell and the modified-release coating.

Hydrophilic molecules are molecules with polar groups. They have a great affinity for water and thus can attract water molecules or be readily soluble in water. In some embodiments, the isolating layer hydrophilic molecule is selected from, but is not limited to, at least one of sucrose, lactose, mannitol, starch and sorbitol.

In some other embodiments, the capsule does not comprise a sealing film between a body and a cap thereof.

In some embodiments, the modified-release coating is a delayed-release coating and/or a controlled-release coating, preferably an enteric coating, and the film-forming agent (1) is selected from the group consisting of an acrylate polymer, a cellulose polymer, a polyvinyl-based polymer and a mixture thereof.

In some other embodiments, the film-forming agent (1) is selected from the group consisting of a copolymer of (meth)acrylic acid and C₁₋₄ alkyl (meth)acrylate, cellulose acetate phthalate, cellulose acetate trimellitate, cellulose acetate succinate, hydroxypropyl methylcellulose phthalate, hydroxypropyl methylcellulose acetate succinate, carboxymethyl ethylcellulose acetate butyrate, polyvinyl acetate phthalate and a mixture thereof.

Further, in certain embodiments, the film-forming agent (1) is a copolymer of (meth)acrylic acid and C₁₋₄ alkyl (meth)acrylate, for example, a copolymer of methacrylic acid and methyl methacrylate. Generally, such copolymers have a molecular weight of about 120,000 to 150,000, preferably about 135,000.

Known poly(methacrylic acid/methyl methacrylate) copolymers include Eudragit L, Eudragit S and Eudragit FS. Eudragit L100-55 is a copolymer of methacrylic acid and ethyl acetate and has a pH of 5.5.

In some embodiments, the film-forming agent (1) is Eudragit L100. In some embodiments, the film-forming agent (1) is Eudragit S100. In some embodiments, the film-forming agent (1) is a mixture of Eudragit L100 and Eudragit S100. In some other embodiments, the modified-release coating comprises Eudragit L100 and Eudragit S100, and the weight ratio of L100 to S100 is 10:1 to 1:1, and may be 10:1, 9:1, 8:1, 7:1, 6:1, 5:1, 4:1, 3:1, 2:1, 1:1, or a value between any two of these numbers, preferably 5:1 to 2:1.

In some other embodiments, the film-forming agent (I) does not comprise cellulose acetate. In some other embodiments, the film-forming agent (I) does not comprise cellulose acetate phthalate.

Further, the modified-release coating comprises at least one of a plasticizer, an anti-adhesive agent, a colorant and a binder, in addition to the aforementioned film-forming agent.

In some embodiments, the plasticizer in the modified-release coating includes, but is not limited to, triethyl citrate, tributyl citrate, dibutyl sebacate, dimethyl phthalate, diethyl phthalate or dibutyl phthalate.

In some embodiments, the binder in the modified-release coating includes, but is not limited to, mannitol, glucose, sucrose, polyethylene glycol, hydroxypropyl methylcellulose, hydroxypropyl cellulose, methylcellulose, glycerol triacetate and water-soluble materials of polyvinyl alcohol. In some other embodiments, the anti-adhesive agent in the modified-release coating is selected from the group consisting of talc, magnesium stearate and glyceryl monostearate.

In another aspect, the modified-release coating comprises 50-80% of the film-forming agent (1) based on the total weight of the modified-release coating (solid). Further, the content of the film-forming agent (1) may be 50%, 52%, 54%, 56%, 58%, 60%, 62%, 64%, 66%, 68%, 70%, 72%, 74%, 76%, 78%, 80%, or a value between any two of these numbers.

In certain embodiments, the modified-release coating comprises 5-15% of the plasticizer based on the total weight of the modified-release coating (solid). Further, the content of the plasticizer may be 5%, 6%, 7%, 8%, 9%, 10%, 11%, 12%, 13%, 14%, 15%, or a value between any two of these numbers.

In certain embodiments, the modified-release coating comprises 5-35% of the anti-adhesive agent based on the total weight of the modified-release coating (solid). Further, the content of the anti-adhesive agent may be 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, or a value between any two of these numbers.

In certain embodiments, the modified-release coating comprises 0-15% of the binder based on the total weight of the modified-release coating (solid). Further, the content of the binder may be 0, 1%, 2%, 3%, 4%, 5%, 6%, 7%, 8%, 9%, 10%, 11%, 12%, 13%, 14%, 15%, or a value between any two of these numbers.

In certain embodiments, the modified-release coating comprises 0-8% of the colorant based on the total weight of the modified-release coating (solid). Further, the content of the colorant may be 0%, 1%, 2%, 3%, 4%, 5%, 6%, 7%, 8%, or a value between any two of these numbers. Further, in some embodiments, the modified-release coating comprises 50-80% of the film-forming agent (1), 5-15% of the plasticizer, 5-35% of the anti-adhesive agent, 0-15% of the binder and 0-8% of the colorant based on the total weight of the modified-release coating.

In another aspect, the capsule shell of the present disclosure may be any suitable material, such as a gelatin shell, a hydroxypropyl methylcellulose shell, a pullulan shell, a gelatin capsule shell, a starch capsule shell or a PVA-based shell. In some embodiments, the capsule shell is a hydroxypropyl methylcellulose shell. In some embodiments, the capsule shell is a starch shell. In some embodiments, the capsule shell is a gelatin shell. The capsule size is not particularly limited and may be any common size, such as 000, 00, 0, 1, 2, 3, 4 or 5.

In addition, the capsule of the present disclosure may be used for being filled with a non-liquid filler including a nutritional ingredient and/or an active drug ingredient. Further, the nutritional ingredient and/or the active drug ingredient is present in the form of powder, granules, pellets and/or mini-tablets.

In some embodiments, the drug is selected from at least one of a drug for treating intestinal diseases, a drug for treating glomerulonephritis, a hypoglycemic drug, an antiviral drug, an anti-Parkinson drug, and a nucleic acid-containing formulation for delivery to intestinal cells.

The drug for treating intestinal diseases is preferably a corticosteroid, an anticholinergic drug and an opioid receptor antagonist; the hypoglycemic drug is preferably insulin and an analog thereof; the antiviral drug is preferably an anti-HIV drug.

In certain embodiments, the drug for treating intestinal diseases includes, but is not limited to, corticosteroids, such as hydrocortisone, budesonide, 5-aminosalicylic acid, cisapride, anticholinergic drugs and opioid receptor antagonists.

In certain embodiments, the hypoglycemic drug includes, but is not limited to, insulin and analogs thereof, and semaglutide.

In certain embodiments, the drug for treating glomerulonephritis includes, but is not limited to, corticosteroids, such as hydrocortisone and budesonide. The anti-Parkinson drug includes, but is not limited to, dopamine and levodopa. In certain embodiments, the aforementioned drugs further comprise at least one pharmaceutically acceptable additional excipient, including but not limited to, fillers, lubricants, glidants, binders and disintegrants.

Further, in some other embodiments, the capsule is filled with a drug that is a corticosteroid, such as budesonide.

In some embodiments, the capsule is filled with a budesonide-containing pharmaceutical composition, which comprises sustained-release components of a drug-containing core, an isolating layer and a sustained-release coating layer (1), wherein the drug-containing core is covered in the isolating layer and the isolating layer is covered in the sustained-release coating layer (1), wherein the drug-containing core comprises a corticosteroid and hydroxypropyl methylcellulose, and the weight ratio of hydroxypropyl methylcellulose to the corticosteroid is at least 2.5:1.

In some embodiments, the weight ratio of lactose to the corticosteroid in the drug-containing core is 2.5:1 to 8:1, and may be 2.6:1, 2.7:1, 2.8:1, 2.9:1, 3.0:1, 3.1:1, 3.2:1, 3.3:1, 3.4:1, 3.5:1, 3.6:1, 3.7:1, 3.8:1, 3.9:1, 4.0:1, 4.1:1, 4.2:1, 4.3:1, 4.4:1, 4.5:1, 4.6:1, 4.7:1, 4.8:1, 4.9:1, 5.0:1, 5.1:1, 5.2:1, 5.3:1, 5.4:1, 5.5:1, 5.6:1, 5.7:1, 5.8:1, 5.9:1, 6.0:1, 6.1:1, 6.2:1, 6.3:1, 6.4:1, 6.5:1, 6.6:1, 6.7:1, 6.8:1, 6.9:1, 7.0:1, 7.1:1, 7.2:1, 7.3:1, 7.4:1, 7.5:1, 7.6:1, 7.7:1, 7.8:1, 7.9:1, 8.0:1, or a value between any two of these numbers.

In some other embodiments, the amount (content) of hydroxypropyl methylcellulose in the drug-containing core makes up 4-20% of the total weight of the drug-containing core, and may be 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, or a value between any two of these numbers. In the embodiments, the amount (content) of hydroxypropyl methylcellulose in the sustained-release components makes up 6-18%, such as 12% or 13%, of the total weight of the sustained-release components.

In the present disclosure, "the total weight of the drug-containing core" or "based on the total weight of the drug-containing core" refers to the numerical range of the amount of the active ingredient or other pharmaceutical excipients calculated on the basis of the weight of the drug-containing core without the isolating layer or the coating.

Further, the isolating layer in the pharmaceutical composition provided by some embodiments comprises hydroxypropyl methylcellulose and does not comprise organic acids, such as citric acid, glutamic acid, lactic acid, tartaric acid, fumaric acid, malic acid and sodium dihydrogen phosphate. In some other embodiments, the isolating layer does not comprise an acid. The "free of" or "substantially free of" means no extra additions. It should be noted that it is sufficient to determine that, for example, the stability of the pharmaceutical composition is not due to the addition of acid. In an exemplary embodiment, the isolating layer of the pharmaceutical composition of the present disclosure needs no additional citric acid, for example, to ensure drug stability.

In some other embodiments, the capsule is filled with a budesonide-containing pharmaceutical composition, which comprises sustained-release components of a drug-containing core, an isolating layer and a sustained-release coating layer (1), wherein the drug-containing core is covered in an acid-containing isolating layer and the isolating layer is covered in the sustained-release coating layer (1). Acid capable of stabilizing the pharmaceutical composition is a weak acid. See CN102088962 for reference, and the relevant contents are incorporated herein for illustrative purposes. In addition, the ingredients and contents of the drug-containing core, the isolating layer and the sustained-release coating in the composition can also be found in CN102088962, and the relevant contents are incorporated herein for illustrative purposes.

In some other embodiments, the capsule is filled with a levodopa-containing pharmaceutical composition, which comprises a levodopa-containing core and a sustained-release coating layer, wherein the levodopa-containing core is covered in the sustained-release coating layer (1).

In another aspect, suitable corticosteroid drugs further include, but are not limited to, alclometasone, beclomethasone, betamethasone, clobetasol, hydrocortisone, dexamethasone, flunisolide, methylprednisolone, mometasone, prednisolone, triamcinolone, budesonide, fluticasone, ciclesonide and fludrocortisone.

In another aspect, the isolating layer further comprises one or more of a film-forming agent (2), a plasticizer, an anti-adhesive agent and a colorant.

In some embodiments, the film-forming agent (2) is selected from the group consisting of hydroxypropyl methylcellulose, hydroxypropyl cellulose, methylcellulose, polyvinylpyrrolidone, polyvinyl alcohol and a mixture thereof.

In certain embodiments, the plasticizer in the isolating layer is selected from one or more of triethyl citrate, tributyl citrate, dibutyl sebacate, dimethyl phthalate and polyethylene glycol.

In some other embodiments, the anti-adhesive agent in the isolating layer is selected from one or more of talc, silicon dioxide, magnesium stearate and glyceryl monostearate, and the colorant is selected from titanium dioxide.

Further, a coating agent for the isolating layer in the capsule provided by some embodiments is selected from Opadry II (product code 85G68918, Colorcon Inc.), which mainly comprises polyvinyl alcohol, talc, polyethylene glycol 3350 and titanium dioxide.

In a certain embodiment, the isolating layer in the capsule comprises Opadry II and sucrose. In some other embodiments, the isolating layer in the capsule comprises Opadry II and lactose. In some other embodiments, the isolating layer in the capsule comprises Opadry II and mannitol. In some other embodiments, the isolating layer in the capsule comprises Opadry II and starch. In some other embodiments, the isolating layer in the capsule comprises Opadry II and sorbitol. In another aspect, the weight ratio of the hydrophilic molecule to the coating agent for the isolating layer in the capsule provided by some embodiments is 1:15 to 10:1, and may be 1:15, 1:14, 1:13, 1:12, 1:11, 1:10, 2:10, 3:10, 4:10, 5:10, 6:10, 7:10, 8:10, 9:10, 1:1, 2:1, 3:1, 4:1, 5:1, 6:1, 7:1, 8:1, 9:1, 10:1, or a value between any two of these numbers, preferably 1:10 to 1:5.

In another aspect, in certain embodiments, the isolating layer is directly applied to the surface of the capsule in an amount of 5-50 mg/cm² based on the surface area of the capsule. In some other embodiments, the isolating layer may be applied in an amount of 5 mg/cm², 6 mg/cm², 7 mg/cm², 8 mg/cm², 9 mg/cm², 10 mg/cm², 11 mg/cm², 12 mg/cm², 13 mg/cm², 14 mg/cm², 15 mg/cm², 16 mg/cm², 17 mg/cm², 18 mg/cm², 19 mg/cm², 20 mg/cm², 21 mg/cm², 22 mg/cm², 23 mg/cm², 24 mg/cm², 25 mg/cm², 26 mg/cm², 27 mg/cm², 28 mg/cm², 29 mg/cm², 30 mg/cm², 31 mg/cm², 32 mg/cm², 33 mg/cm², 34 mg/cm², 35 mg/cm², 36 mg/cm², 37 mg/cm², 38 mg/cm², 39 mg/cm², 40 mg/cm², 41 mg/cm², 42 mg/cm², 43 mg/cm², 44 mg/cm², 45 mg/cm², 46 mg/cm², 47 mg/cm², 48 mg/cm², 49 mg/cm², 50 mg/cm², or a value between any two of these numbers based on the surface area of the capsule.

In certain embodiments, the modified-release coating is directly applied onto a first coating in an amount of 5-20 mg/cm² based on the surface area of the capsule. In some other embodiments, the modified-release coating may be applied in an amount of 5 mg/cm², 6 mg/cm², 7 mg/cm², 8 mg/cm², 9 mg/cm², 10 mg/cm², 11 mg/cm², 12 mg/cm², 13 mg/cm², 14 mg/cm², 15 mg/cm², 16 mg/cm², 17 mg/cm², 18 mg/cm², 19 mg/cm² or 20 mg/cm².

In some embodiments, the isolating layer coating increases the weight of the capsule shell by 3-20%, and the weight increase may be 3%, 4%, 5%, 6%, 7%, 8%, 9%, 10%, 11%, 12%, 13%, 14%, 15%, 16%, 17%, 18%, 19%, 20%, or a value between any two of these numbers. In some embodiments, the concentration of the isolating layer solution is 4%. In some embodiments, the amount of the isolating layer in a unit formula is 1.6 mg-2.0 mg, which is sufficient to provide isolation without affecting the granule content, so that granule aggregation is avoided during coating.

In another aspect, the amount of the sustained-release material is also a critical factor. In some embodiments, the modified-release coating increases the weight of the capsule comprising the isolating layer by 3-20%, and the weight increase may be 3%, 4%, 5%, 6%, 7%, 8%, 9%, 10%, 11%, 12%, 13%, 14%, 15%, 16%, 17%, 18%, 19%, 20%, or a value between any two of these numbers.

In some other embodiments, the capsule further comprises one or more additional coatings.

In certain embodiments, the capsule releases substantially no drug in gastric acid, but in the ileum and caecum.

### Terminology:

As used herein, "intestinal tract" refers to the digestive tract from the pylorus of the stomach to the anus. The mammalian intestines include the small intestine and the large intestine. The human small intestine includes the duodenum, the jejunum and the ileum; the large intestine includes the cecum and the colon.

As used herein, "substantially release in the intestinal tract" generally means that after oral administration, the pharmaceutical composition barely releases the active substance until it arrives in the intestinal tract. For example, no more than 15%, preferably no more than 10%, and most preferably no more than 5% of the drug is released from the pharmaceutical composition before arrival in the intestinal tract. Drug release generally begins in the small intestine. Drug release may also be delayed until the pharmaceutical composition arrives at a specific part of the intestine, for example, the duodenum, the colon, the ileum or the caecum.

The values in the present disclosure are instrument measurements or calculated values after instrument measurement, and have a certain degree of error. Generally, ±10% falls within a reasonable error range. It is certainly necessary to consider the context in which the value is used, for example, for the content of the active ingredient, the error range of the value after the measurement shall not exceed ±10%, and may be ±9%, ±8%, ±7%, ±6%, ±5%, ±4%, ±3%, ±2% or ±1%, preferably ±5%.

In the present disclosure, "free of" or "substantially free of" means no extra additions. It should be noted that it is sufficient to determine that, for example, the stability of the pharmaceutical composition is not due to the addition of acid. In an exemplary embodiment, the isolating layer of the pharmaceutical composition of the present disclosure needs no additional citric acid, for example, to ensure drug stability.

### HPLC analysis conditions:

Column: octadecylsilane bonded silica gel as a filler (Agilent Eclipse XDB-C18, 4.6 mm × 150 mm, 3.5 µm); mobile phase: potassium phosphate (pH 4.0)-acetonitrile (30:70); detection wavelength: 240 nm. The dissolution of each capsule is calculated from peak areas using the external standard method.

### DETAILED DESCRIPTION

The specific examples of the present disclosure are shown below and are intended to further describe rather than limit the present disclosure. Any technical solution equivalent to the present disclosure falls within the scope of the present disclosure.

### Example 1

**Table 1**

| | Formulation 1 | Weight |
|---|---|---|
| Drug-containing core | Sucrose sphere | 200 g |
| | Budesonide | 6 g |
| | Hydroxypropyl methylcellulose | 30 g |
| | Talc | 4.5 g |
| Isolating layer | Hydroxypropyl methylcellulose | 12 g |
| | Talc | 1.8 g |
| Sustained-release coating layer | Surelease | 60 g |
| | Hydroxypropyl methylcellulose | 3.75 g |

### 1) Drug-containing solution

30 g of hydroxypropyl methylcellulose (HPMC E5 LV), 4.5 g of talc and 6 g of budesonide were weighed out according to the formula and dispersed in purified water, and the mixture was well stirred to form a homogenous suspension for later use.

### 2) Isolating layer solution

12 g of hydroxypropyl methylcellulose (HPMC E5 LV) and 1.8 g of talc were weighed out and added to purified water, and the mixture was well stirred to form a homogenous suspension for later use.

### 3) Drug coating and isolating layer

200 g of sucrose spheres (0.71-0.85 mm, Hangzhou Gaocheng) was loaded into the coating chamber of a fluidized bed (FLZB-0.5, Chanse Mechatronics) with the inlet air temperature set to 55 °C and the atomization pressure set to 1.2 bar, and the aforementioned drug-containing solution was applied at a rate of 8 g/min. After the application, drying was performed, and the coated pellets were granulated.

Then the isolating layer solution was applied at a rate of about 8 g/min. After the application, drying was performed, and the coated pellets were granulated.

### 4) Sustained-release coating layer

3.75 g of hydroxypropyl methylcellulose (HPMC E5 LV) was dispersed in 40 g of purified water, and 60 g of an aqueous dispersion of Surelease was added. The mixture was well stirred to form a controlled-release layer coating solution.

The controlled-release layer coating solution was applied onto the granules (or drug-loaded spheres) obtained in step 3 at a rate of about 8 g/min in a fluidized bed (inlet air temperature: 55 °C; atomization pressure: 1.2 bar). After fluidization and drying, the coated pellets were granulated.

The granules in step 2 were coated in a fluidized bed to obtain sustained-release pellet granules.

### 5) Filling-coating

Preparing capsule shell isolating layer solutions: the excipients in Table 2 were dissolved in 95% ethanol and/or purified water to form isolating layer solutions with different compositions for later use.

**Table 2**

| | Formula 2 | Comparative formula 2-1 | Comparative formula 2-2 | Comparative formula 2-3 |
|---|---|---|---|---|
| Opadry I | / | 75 g | / | / |
| Opadry II | 100g | / | 100 g | 100 g |
| Sucrose | 10 g | / | / | / |
| Methylcellulose (MC) | / | 45 g | 10 g | / |
| Purified water | 146 g | 880 g | 146 g | 146 g |
| 95% ethanol | 460 g | / | 460 g | 460 g |

Preparing an enteric coating solution: 12 g of triethyl citrate was dissolved in 1844 g of 95% ethanol, 90 g of Eudragit L100 and 30 g of Eudragit S100 were added and dissolved, and then 24 g of talc was added and uniformly dispersed for later use.

The budesonide-containing sustained-release pellets (formulation 1) obtained above were loaded into hydroxypropyl methylcellulose capsules (manufacturer: Qingdao Yiqing).

The isolating layer solutions were applied to the surface of the capsules filled with the sustained-release pellets of budesonide at a rate of about 8 g/min in a fluidized bed. After the application, the capsules were dried.

Then the enteric coating solution was applied at a rate of about 20 g/min (coating amount: 10 mg/cm²). After the application, the capsules were dried to obtain budesonide enteric-coated capsules.

Samples of the aforementioned formulation 1 were placed in sealed aluminum foil bags and stored in a 60 °C thermostatic incubator and a 40 °C thermostatic incubator, respectively. The impurity content of the budesonide pellets was determined by high performance liquid chromatography, and the increases in impurity content in 4 weeks relative to day 0 were calculated. The related data are shown in Table 3.

**Table 3**

| Time | | Formulation 1 (%) |
|---|---|---|
| Initial total impurities (day 0) | | 0.33 |
| 1-week total impurities | 40°C | 0.39 |
| | 60°C | 0.47 |
| 2-week total impurities | 40°C | 0.42 |
| | 60°C | 0.47 |
| 4-week total impurities | 40°C | 0.41 |
| | 60°C | 0.49 |
| Level of total impurities | 40°C | 0.08 |
| | 60°C | 0.16 |

The dissolution test method of Chinese Pharmacopoeia (the basket method) was used to test the release of the enteric-coated capsule formulation 2 and comparative formulations 2-1 to 2-3 obtained according to the aforementioned formula 2 and comparative formulas 2-1 to 2-3 (900 mL of dissolution medium was used. The formulations were tested first in media having a pH of 1.0 for 2 h, and then placed into phosphate buffer solutions having a pH of 6.8. The temperature of the dissolution medium was 37±0.5 °C, the speed was 100 rpm, and HPLC analysis was performed). The test results are shown in Table 4.

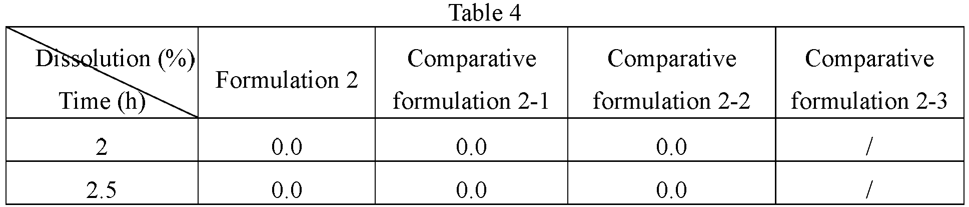

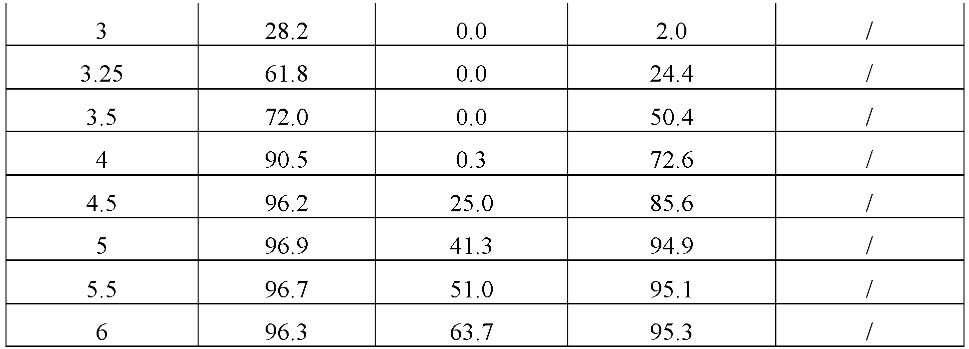

The results show that: after comparative formulation 2-1 (Opadry I/MC) was left under simulated gastric pH 1 conditions for 2 h, some cracks were formed in the middle of the capsule, and acid seeped in through the cracks; after the formulation was transferred to a pH 6.8 solution, the acid that seeped in affected the sustained release of the sustained-release material Surelease, slowing down the release and resulting in an incomplete release; finally, the drug could not be effectively absorbed in the intestinal tract.

Formulation 2 (Opadry II/sucrose) was left under simulated gastric pH 1 conditions for 2 h and then transferred to a pH 6.8 solution. The capsule of formulation 2 broke faster than that of comparative formulation 2-2 (Opadry II/MC), and the pellets contained in the capsule dissolved faster and to a greater extent.

In the process of preparing comparative formulation 2-3 (Opadry II), after the capsules were coated with Opadry II, cracks were formed in the place where the body and cap of capsules were joined in the subsequent drying process. The presence of the cracks increases the potential risk that the capsules will break early *in vivo*, affecting their site-specific sustained release.

### Example 2

### 1) Preparation of coating solutions

Preparing capsule shell isolating solutions: the excipients in Table 5 were dissolved in 95% ethanol and purified water to form isolating layer solutions with different compositions for later use.

**Table 5**

| | Formula 3 | Formula 4 | Formula 5 | Formula 6 | Formula 7 |
|---|---|---|---|---|---|
| Opadry II | 100g | 100 g | 100 g | 100 g | 100 g |
| Sucrose | 10 g | / | / | 20 g | 33.33 g |
| Mannitol | / | 10 g | / | / | / |
| Lactose | / | / | 10 g | / | / |
| Purified water | 146 g | 146 g | 146 g | 146 g | 146 g |
| 95% ethanol | 460 g | 460 g | 460 g | 460 g | 460 g |

Preparing an enteric coating solution: 12 g of triethyl citrate was dissolved in 1844 g of 95% ethanol, 90 g of Eudragit L100 and 30 g of Eudragit S100 were added and dissolved, and then 24 g of talc was added and uniformly dispersed for later use.

### 2) Filling-coating

The budesonide-containing sustained-release pellets (formulation 1) obtained above were loaded into hydroxypropyl methylcellulose capsules (manufacturer: Qingdao Yiqing).

The isolating layer solutions were applied to the surface of the capsules of budesonide-containing sustained-release pellets at a rate of about 8 g/min in a fluidized bed. After the application, the capsules were dried.

Then the enteric coating solution was applied at a rate of about 20 g/min (coating amount: 12 mg/cm²). After the application, the capsules were dried to obtain budesonide enteric-coated capsules.

The dissolution test method of Chinese Pharmacopoeia (the basket method) was used to test the release of the enteric-coated capsule formulations 3 to 7 obtained according to formula 3 to formula 7 (900 mL of dissolution medium was used. The formulations were tested first in media having a pH of 1.0 for 2 h, and then placed into phosphate buffer solutions having a pH of 6.8. The temperature of the dissolution medium was 37±0.5 °C, the speed was 100 rpm, and HPLC analysis was performed). The test results are shown in Table 6.

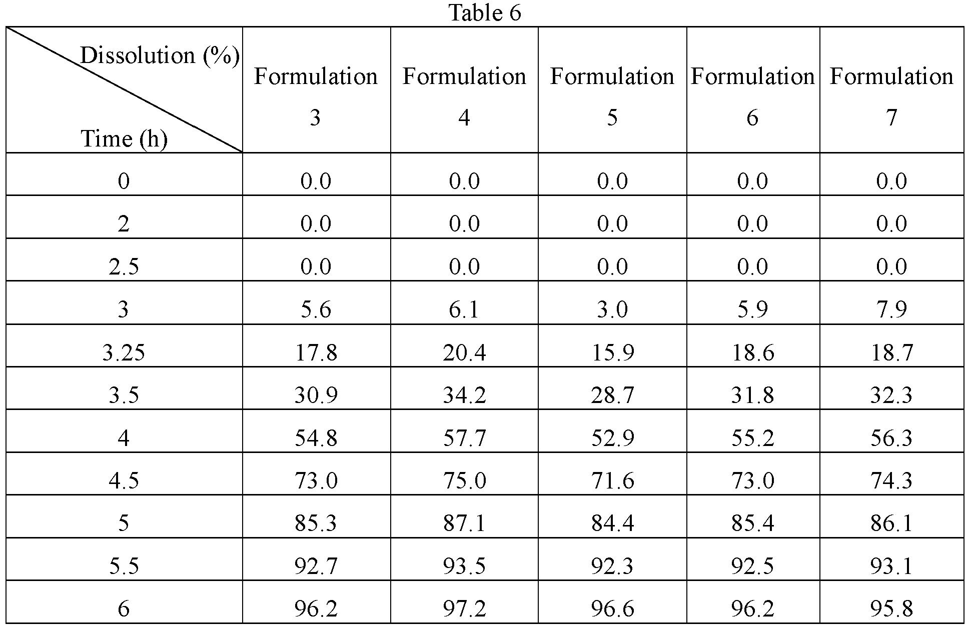

The results show that: the capsule shells with the isolating layer comprising such hydrophilic molecules as mannitol, lactose and sucrose showed similar *in*-*vitro* releases, and can ensure that the release of the capsule content is not affected. Releases were the same when the weight ratio of Opadry II to sucrose was 10: 1, 5: 1 and 3: 1.

### Example 3: Levodopa Sustained-Release Pellet Capsules

### 1) Preparation of drug-containing pellets:

540 g of levodopa drug substance, 240 g of microcrystalline cellulose, 73 g of croscarmellose sodium and 28 g of polyvinylpyrrolidone were weighed out and together subjected to extrusion-spheronization to prepare drug-containing pellets.

### 2) Preparation of sustained-release pellets

70 g of cellulose acetate was weighed out and dispersed in isopropanol, and then 35 g of povidone S630 was added and dissolved. The mixture was stirred for later use. The above drug-containing pellets were coated in a fluidized bed to obtain sustained-release pellets.

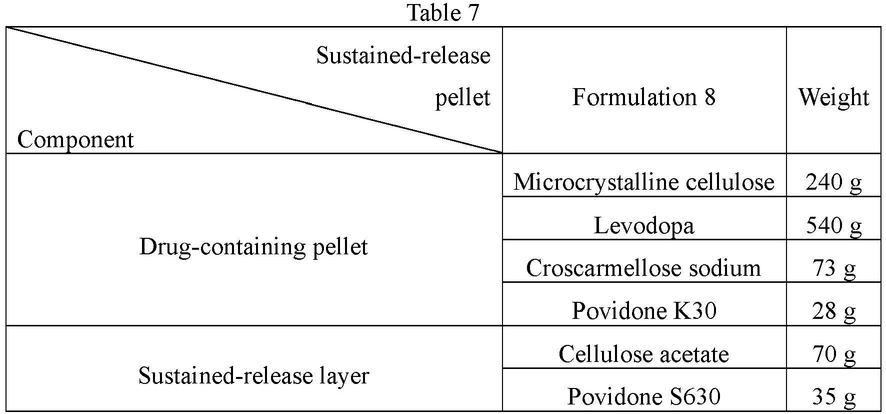

### 3) Levodopa enteric-coated capsule

### 3.1. Preparation of coating solution

Preparing a capsule shell isolating solution: 146 g of purified water and 460 g of 95% ethanol were mixed, and 100 g of Opadry II and 10 g of sucrose were added and well dispersed for later use.

Preparing an enteric coating solution: 12 g of triethyl citrate was dissolved in 95% ethanol (w/w), 120 g of Eudragit L100 was added and dissolved, and talc was added and uniformly dispersed for later use.

### 3.2) Filling-coating

The levodopa-containing sustained-release pellets (formulation 8) obtained above were loaded into HPMC capsules #0, and the isolating layer solution was applied to the surface of the capsule shells at a rate of about 8 g/min (coating amount: 25 mg/cm²) in a fluidized bed. After the application, the capsules were dried.

Then the enteric coating solution was applied at a rate of about 20 g/min (coating amount: 12 mg/cm², a 25% increase in weight). After the application, the capsules were dried to obtain levodopa enteric-coated capsules.

The dissolution test method of Chinese Pharmacopoeia (the basket method) was used to test the release of the levodopa enteric-coated capsule formulation 9 obtained from formulation 8 (900 mL of dissolution medium was used. The formulation was tested first in a medium having a pH of 1.0 for 2 h, and then placed into a phosphate buffer solution having a pH of 6.8. The temperature of the dissolution medium was 37±0.5 °C, the speed was 100 rpm, and HPLC analysis was performed). The test results are shown in Table 8.

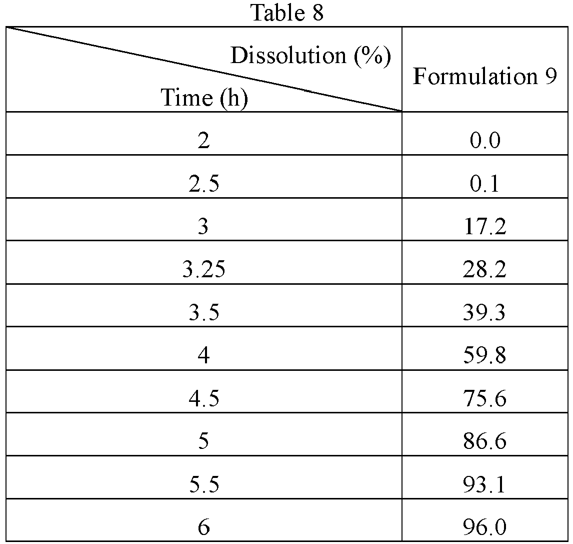

The results show that: the enteric-coated capsule formulation can substantially achieve no release of levodopa under simulated gastric pH 1 conditions in 2 h; after being transferred to a pH 6.8 medium, the formulation released up to 96% over 6 h; the release rate was completely the same as that of the levodopa sustained-release pellets, and levodopa could be completely released, which indicate that the sustained-release component is not affected by the enteric coating at all.

### Comparative Example 1A

Sustained-release pellets were prepared according to the formula in Table 9 using the method of Example 1 and loaded into enteric-coated capsules (Suzhou Capsugel Ltd.) to obtain enteric-coated sustained-release capsule formulation 11.

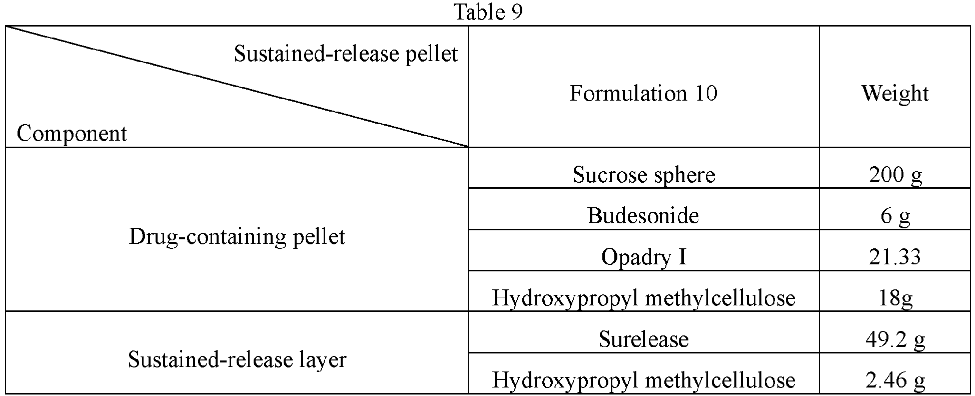

The dissolution test method of Chinese Pharmacopoeia (the basket method) was used to test the release of the budesonide enteric-coated capsule formulation 11 obtained from formulation 10 (900 mL of dissolution medium was used. The formulation was tested first in a medium having a pH of 1.0 for 2 h, and then placed into a phosphate buffer solution having a pH of 6.8. The temperature of the dissolution medium was 37±0.5 °C, the speed was 100 rpm, and HPLC analysis was performed). The test results are shown in Table 10.

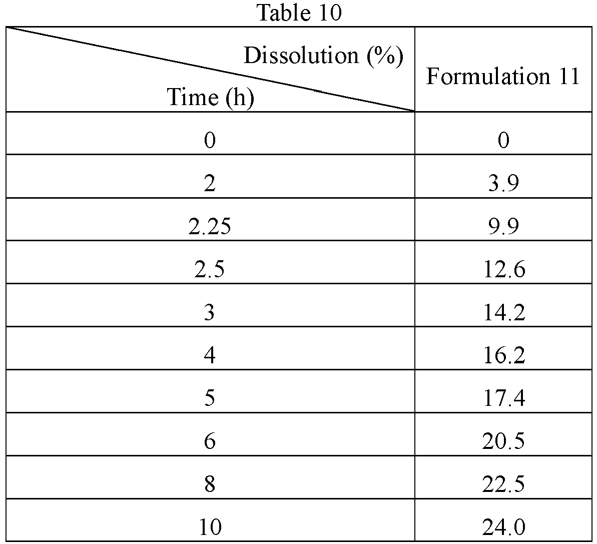

After the enteric-coated capsule formulation was left under simulated gastric pH 1 conditions for 2.0 h, the surface of the capsule wrinkled, and part of the drug was released. After being transferred to a pH 6.8 medium, the formulation released 12.6% over 0.5 h. The overall release was slow and incomplete after the formulation was transferred to the simulated intestinal condition, probably due to acid seeping into the capsule.

### Comparative Example 1B

Sustained-release pellet formulation 12 was prepared according to the formula in Table 11 using the method of Example 1 and loaded into HPMC capsules #0 and starch capsules #0 (Hunan Er-Kang). The gaps between the body and cap of capsules were sealed with a mixed solution of PVA and HPMC. Then the capsules were enteric-coated to obtain enteric-coated sustained-release capsule formulations 13 and 14.

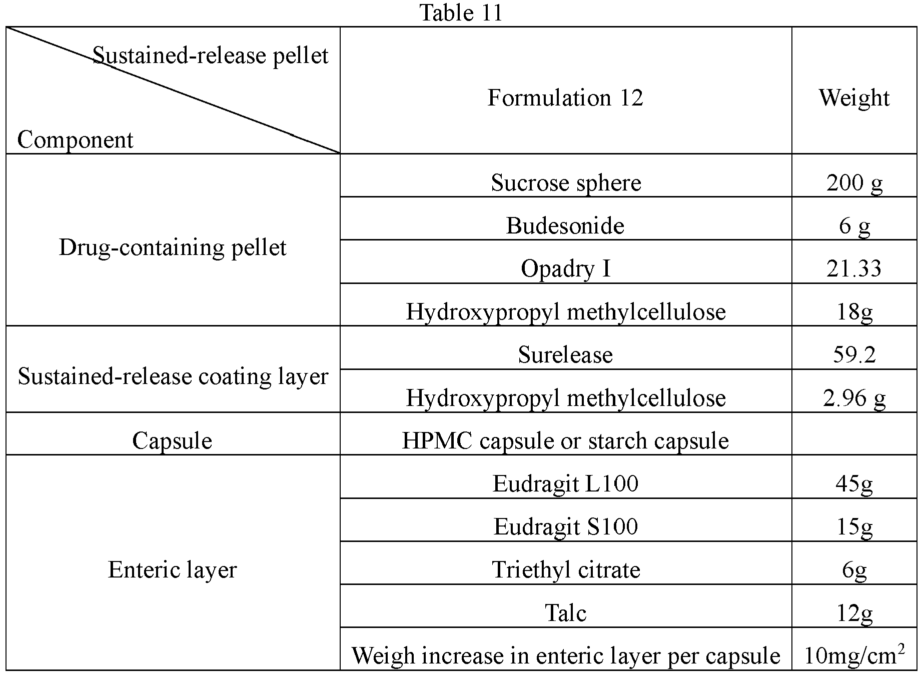

The dissolution test method of Chinese Pharmacopoeia (the basket method) was used to test the release of the budesonide enteric-coated capsule formulations 13 and 14 obtained from formulation 12 (900 mL of dissolution medium was used. The formulations were tested first in media having a pH of 1.0 for 2 h, and then placed into phosphate buffer solutions having a pH of 6.8. The temperature of the dissolution medium was 37±0.5 °C, the speed was 100 rpm, and HPLC analysis was performed). The test results are shown in Table 12.

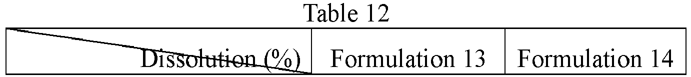

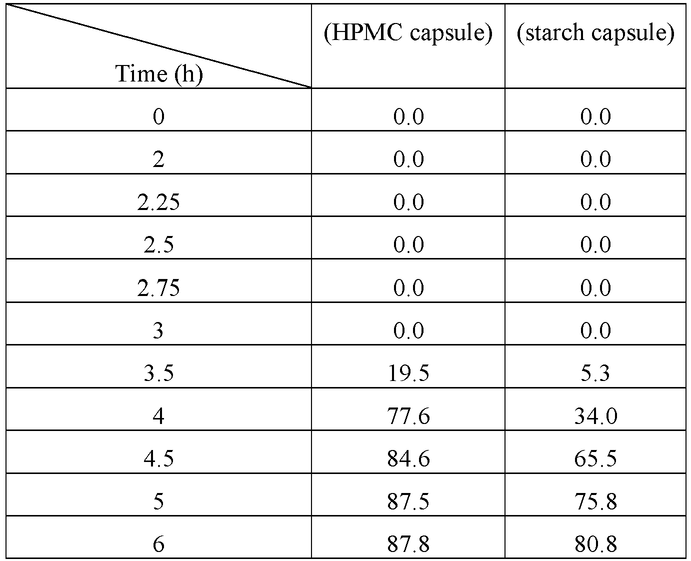

Compared with the sustained-release pellets not loaded into capsules, formulation 13 and formulation 14 can achieve intestinal tract-specific release; however, the drug release rate and the cumulative release amount in the intestinal tract are low. It is expected that such formulations will not achieve effective release within a limited retention time in the intestinal tract, thereby affecting drug bioavailability.

### Comparative Example 1C-1E

Budesonide sustained-release pellets prepared using the method of Example 1 were loaded into HPMC capsules #0, and the capsules were band-sealed with HPMC and then enteric-coated to obtain enteric-coated sustained-release capsule formulation 15.

Budesonide sustained-release pellets prepared using the method of Example 1 were loaded into HPMC capsules #0. An HPMC coating was used as an isolating layer or a pre-coating layer. Then the capsules were enteric-coated to obtain enteric-coated sustained-release capsule formulation 16.

Budesonide sustained-release pellets prepared using the method of Example 1 were loaded into HPMC capsules #0, and the capsules were enteric-coated to obtain enteric-coated sustained-release capsule formulation 17. The gaps between the body and cap of capsules were not band-sealed with any gelatin or HPMC, and no pre-coating layer or isolating layer was present between the capsule shell and the enteric coating.

The formulation 15, formulation 16 and formulation 17 prepared above were separately left under simulated gastric pH 1 conditions for 2 h. The appearance of formulation 15 capsules remained intact, and pellets could be heard rustling inside the capsules, which indicate that acid did not seep into the capsules. Three of the six finished capsules of formulation 16 broke in the middle, and three capsules had noticeable cracks at the gaps. All of the six finished capsules of formulation 17 broke in the middle.

The dissolution test method of Chinese Pharmacopoeia (the basket method) was used to test the release of formulation 15 (900 mL of dissolution medium was used. The formulation was tested first in a medium having a pH of 1.0 for 2 h, and then placed into a phosphate buffer solution having a pH of 6.8. The temperature of the dissolution medium was 37±0.5 °C, the speed was 100 rpm, and HPLC analysis was performed). The test results are shown in Table 13.

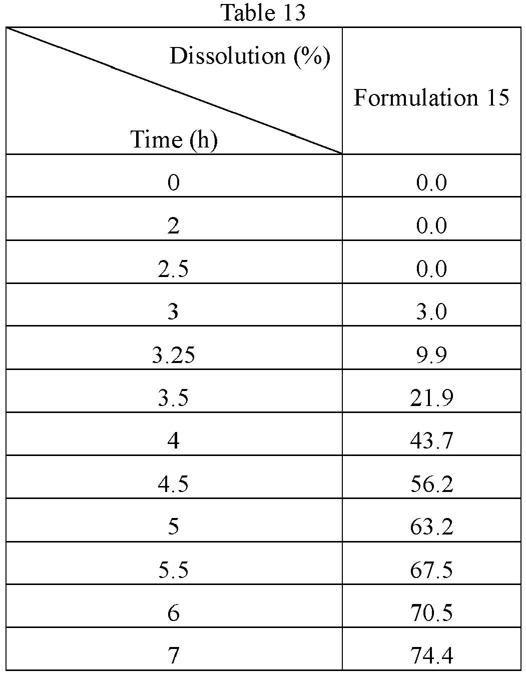

### Example 4

A sustained-release pellet formulation was prepared according to the formula in Table 14 using the method of Example 1 and loaded into HPMC capsules #2, and the capsules were coated with different isolating layers. The gaps between the body and cap of capsules needed no additional adhesive sealing or band sealing. Then the capsules were enteric-coated to obtain enteric-coated sustained-release capsule formulations 18 and 19.

**Table 14**

| Component | Formula component | Formulation 18 | Formulation 19 |
|---|---|---|---|
| Drug-containing sphere | Sucrose sphere | 700g | 600g |
| | Opadry I | 74.55g | / |
| | HPMC E5 | 63g | 90g |
| | Budesonide | 21g | 18g |
| | Talc | 18.9g | 10. 8g |
| Isolating layer | HPMC E5 | / | 36g |
| | Talc | / | 10.8g |
| Alkali-containing ethylcellulose layer | Surelease | 183.75g | 135g |
| | Opadry I | 11.48g | / |
| | HPMC E5 | / | 5.06g |

| Hand-filled capsule | | HPMC capsule #2 | HPMC capsule #2 |
|---|---|---|---|
| Isolating layer (1) | Sucrose | / | 7.6g |
| | Opadry II | 540g | 76g |
| | Weigh increase in isolating layer 1 per capsule | 28.5%(19.4mg/cm²) | 26.2%(18.2mg/cm²) |
| Isolating layer (2) | Opadry I | 150g | / |
| | Weigh increase in isolating layer 2 per capsule | 7.2%(6.3mg/cm²) | / |
| Enteric layer | Eudragit L100 | 126g | 45g |
| | Eudragit S100 | 42g | 15g |
| | Triethyl citrate | 16.8g | 6g |
| | Talc | 33.6g | 12g |
| | Weigh increase in enteric layer per capsule | 12.0%(12.6mg/cm²) | 11.5%(10.1mg/cm²) |

The dissolution test method of Chinese Pharmacopoeia (the basket method) was used to test their release (900 mL of dissolution medium was used. The formulations were tested first in media having a pH of 1.0 for 2 h, and then placed into phosphate buffer solutions having a pH of 6.8. The temperature of the dissolution medium was 37±0.5 °C, the speed was 100 rpm, and HPLC analysis was performed). The test results are shown in Table 15.

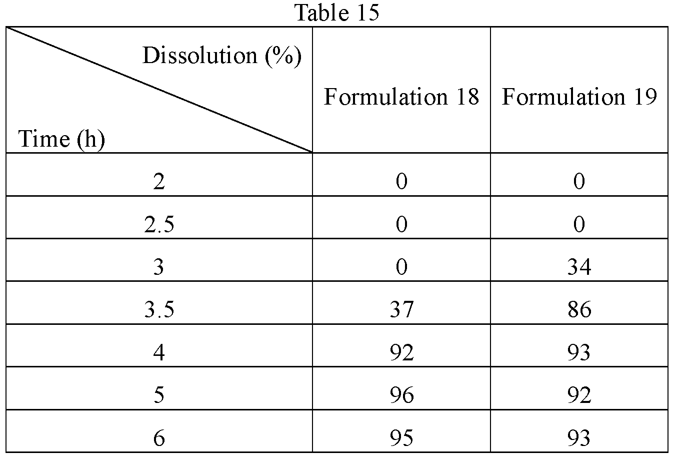

Given the cracks present in the place where the body and cap of capsules were joined in the process of preparing comparative formulation 2-3, two isolating layer coatings were formed for formulation 18 to eliminate cracks in capsules; however, multiple isolating layers delayed capsule breaking, such that capsules could not effectively break to release the drug inside after arriving in the intestinal tract, affecting site-specific drug release and thereby drug bioavailability and efficacy. Accordingly, formulation 19 did not have cracks in the Opadry II-only coating and could effectively break to release the drug inside after arriving in the intestinal tract, achieving the intestinal tract-specific sustained release of the content.

### Example 5

### 1) Filling-coating

Preparing capsule shell isolating solutions: the excipients in Table 16 were dissolved in 95% ethanol and purified water to form isolating layer solutions with different compositions for later use.

**Table 16**

| | Formula 2 | Formula 2A | Formula 2B |
|---|---|---|---|
| Opadry II | 100g | 100 g | 100 g |
| Sucrose | 10 g | / | / |
| Hydroxypropyl cellulose (HPC) | / | 10 g | / |
| Hydroxypropyl methylcellulose (HPMC) | / | / | 10g |
| Purified water | 146 g | 146 g | 146 g |
| 95% ethanol | 460 g | 460 g | 460 g |

Preparing an enteric coating solution: 12 g of triethyl citrate was dissolved in 1844 g of 95% ethanol, 90 g of Eudragit L100 and 30 g of Eudragit S100 were added and dissolved, and then 24 g of talc was added and uniformly dispersed for later use.

### 2) Filling-coating

Budesonide-containing sustained-release pellet granules (prepared according to steps 1-4 of Example 1) were loaded into hydroxypropyl methylcellulose capsules (manufacturer: Qingdao Yiqing).

The isolating layer solutions were applied to the surface of the capsules of budesonide-containing sustained-release pellets at a rate of about 8 g/min in a fluidized bed. After the application, the capsules were dried.

Then the enteric coating solution was applied at a rate of about 20 g/min (coating amount: 10 mg/cm²). After the application, the capsules were dried to obtain budesonide enteric-coated capsules.

The dynamic dissolution method was used to test the release of the enteric-coated capsule formulations 2 to 2B obtained according to formula 2 to formula 2B (900 ML of dissolution medium was used. The formulations were tested first in media having a pH of 1.0 for 2 h, and then placed into Hank's+pre-krabs solutions. The temperature of the dissolution medium was 37±0.5 °C, the speed was 100 rpm, and HPLC analysis was performed (see Journal of Drug Delivery Science and Technology, 2015, 25: 36-42.)). The test results are shown in Table 17.

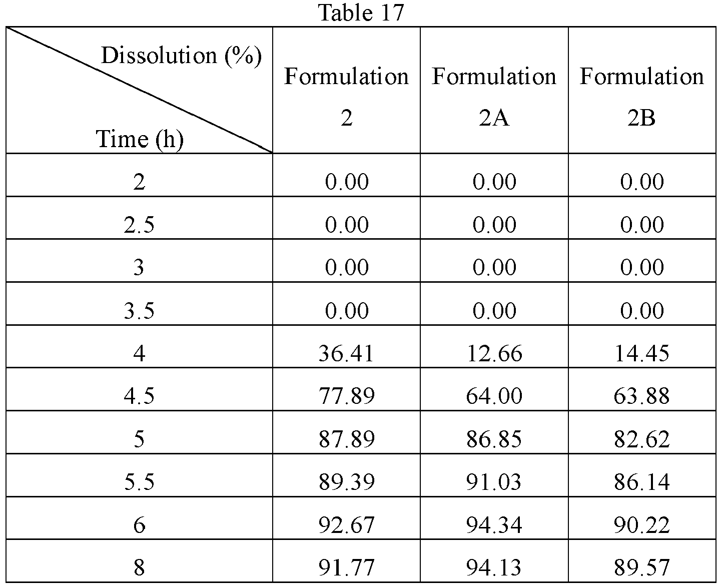

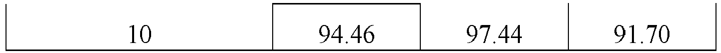

The results show that: the capsule shell with the isolating layer comprising such a hydrophilic molecule as sucrose achieved a faster release *in vitro* in an early stage than the capsule shells with the isolating layers comprising hydroxypropyl cellulose and hydroxypropyl methylcellulose, and it can be clearly observed from their release that the capsule shell with the isolating layer comprising such a hydrophilic molecule as sucrose dissolved faster and formed turbidity in the dissolution cup faster than the capsule shell with the isolating layer comprising hydroxypropyl methylcellulose. This can ensure that the capsule content release is not affected.

### Example 6

An isolating coating solution (formula 2 in Table 16) was prepared according to Example 5. The budesonide-containing sustained-release pellets (formulation 1) obtained above were loaded into hydroxypropyl methylcellulose capsules (manufacturer: Qingdao Yiqing). The isolating layer solution was applied to the surface of the capsules of budesonide-containing sustained-release pellets at a rate of about 8 g/min in a fluidized bed until different weight increases were achieved (as formula 2C and formula 2D in Table 18), and the capsules were dried.

**Table 18**

| | Formula 2 | Formula 2C | Formula 2D |
|---|---|---|---|
| Weight increase per unit area of isolating layer coating | 20mg/cm² | 30 mg/cm² | 45 mg/cm² |

Then the enteric coating solution was applied at a rate of about 20 g/min (coating amount: 10 mg/cm²). After the application, the capsules were dried to obtain budesonide enteric-coated capsules.

The dynamic dissolution method was used to test the release of the enteric-coated capsule formulation 2, formulation 2C and formulation 2D obtained according to formula 2, formula 2C and formula 2D (900 ML of dissolution medium was used. The formulations were tested first in media having a pH of 1.0 for 2 h, and then placed into Hank's+pre-krabs solutions. The temperature of the dissolution medium was 37±0.5 °C, the speed was 100 rpm, and HPLC analysis was performed (see Journal of Drug Delivery Science and Technology, 2015, 25: 36-42.)). The test results are shown in Table 19.

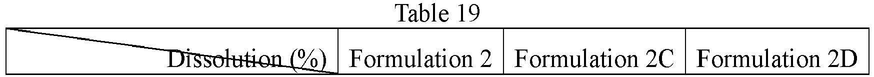

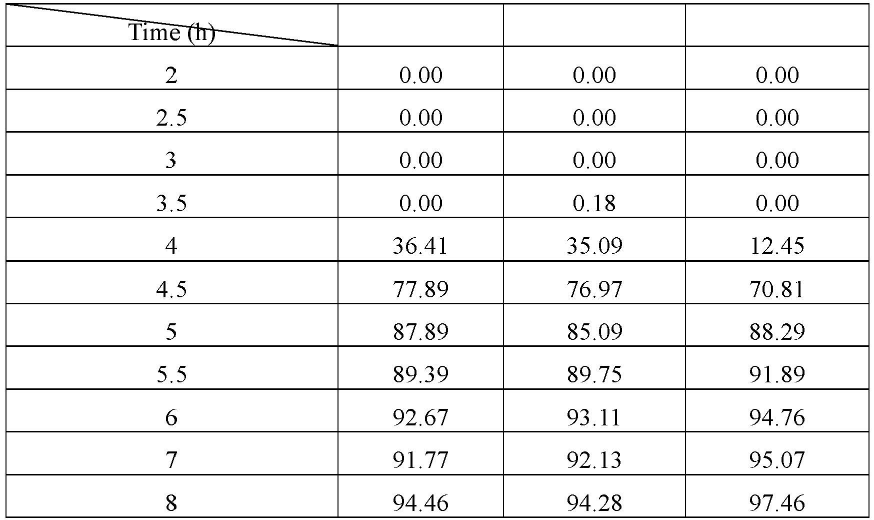

The results show that: as the thickness of the isolating layer coating of the capsule (or the coating per unit surface area) increases, there is a downward trend in the *in vitro* release rate of formulation 2D in an early stage compared to formulation 2. Therefore, an excessively thick isolating layer coating may affect the release rate and may eventually lead to inefficient drug absorption in the intestinal tract.

### Example 7

### 1) Filling-coating

Preparing capsule shell isolating solutions: the excipients in Table 20 were dissolved in absolute ethanol and purified water to form isolating layer solutions with different compositions for later use.

**Table 20**

| | Formula 2E | Formula 2F | Formula 2G |
|---|---|---|---|
| Opadry II | 100g | | 100 g |
| Opadry I | | 100 g | |
| Sucrose | 10 g | / | 10 g |
| Hydroxypropyl cellulose (HPC) | / | 10 g | / |
| Purified water | 117.6 g | 117.6 g | 117.6 g |
| Absolute ethanol | 426.2 g | 426.2 g | 426.2 g |

Preparing an enteric coating solution: 13.4 g of triethyl citrate was dissolved in a mixture of 1489.4 g of absolute ethanol and 78.39 g of purified water, 100.5 g of Eudragit L100 and 33.5 g of Eudragit S100 were added and dissolved, and then 26.8 g of talc was added and uniformly dispersed for later use.

### 2) Filling-coating

Budesonide-containing sustained-release pellet granules (prepared using the method for formulation 19 of Example 4) were loaded into hydroxypropyl methylcellulose capsules (manufacturer: Qingdao Yiqing).

The gaps between the body and cap of capsules were sealed with HPMC solution, and then isolating coatings of Opadry II and sucrose were formed. Then the capsules were enteric-coated to obtain enteric-coated formulation 2G. The gaps between the body and cap of the capsules of enteric-coated formulation 2E and enteric-coated formulation 2F were not sealed with HPMC solution.

The isolating layer solutions were applied to the surface of the capsules of budesonide-containing sustained-release pellets at a rate of about 8 g/min in a fluidized bed. After the application, the capsules were dried.

Then the enteric coating solution was applied at a rate of about 20 g/min. After the application, the capsules were dried to obtain budesonide enteric-coated capsules.

The dissolution test method of Chinese Pharmacopoeia (the basket method) was used to test their release (900 mL of dissolution medium was used. The formulations were tested first in media having a pH of 1.0 for 2 h, and then placed into phosphate buffer solutions having a pH of 6.8. The temperature of the dissolution medium was 37±0.5 °C, the speed was 100 rpm, and HPLC analysis was performed). The test results are shown in Table 21.

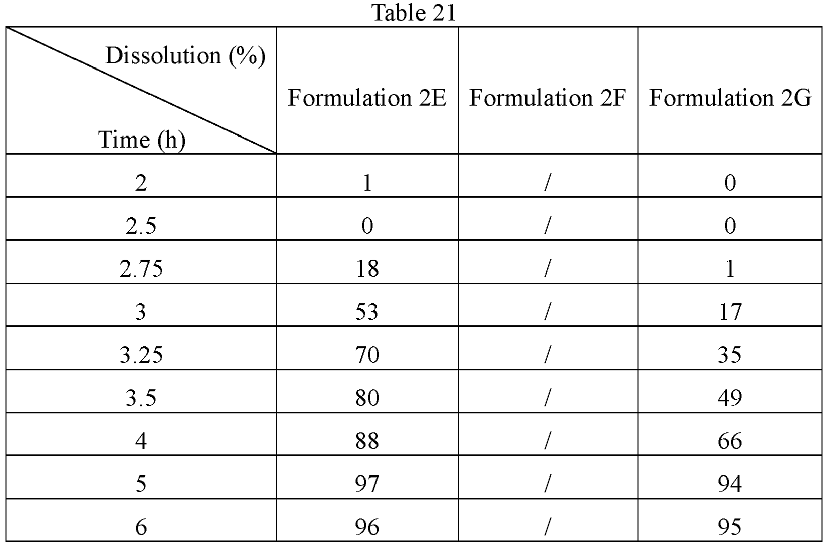

The results show that: formulation 2G, which was formed by sealing the gaps between the body and cap of capsules with HPMC solution first and then forming the isolating layer, showed a significantly slower release *in vitro* than formulation 2E, and it can be seen from its release that capsule disintegration was delayed.

In the process of preparing formulation 2F, in coating capsules with Opadry I/HPC, cracks were formed in the place where the body and cap of capsules were joined. The presence of the cracks increases the potential risk that the capsules will break early *in vivo*, affecting their site-specific sustained release.

## Claims

1. A capsule comprising a non-liquid filler and comprising a modified-release coating, wherein a capsule shell is covered in the modified-release coating, and an isolating layer comprising a hydrophilic molecule is arranged between the capsule shell and the modified-release coating, wherein the hydrophilic molecule is preferably at least one of sucrose, lactose, starch and mannitol.

2. The capsule according to claim 1, wherein the capsule does not comprise a sealing film between a body and a cap thereof.

3. The capsule according to claim 1 or 2, wherein the modified-release coating is a delayed-release coating and/or a controlled-release coating, preferably an enteric coating, and a film-forming agent (1) is selected from the group consisting of an acrylate polymer, a cellulose polymer, a polyvinyl-based polymer and a mixture thereof.

4. The capsule according to claim 3, wherein the film-forming agent (1) is selected from the group consisting of a copolymer of (meth)acrylic acid and C₁₋₄ alkyl (meth)acrylate, cellulose acetate phthalate, cellulose acetate trimellitate, cellulose acetate succinate, hydroxypropyl methylcellulose phthalate, hydroxypropyl methylcellulose acetate succinate, carboxymethyl ethylcellulose acetate butyrate, polyvinyl acetate phthalate and a mixture thereof.

5. The capsule according to any one of claims 1-4, wherein the film-forming agent (1) is selected from the group consisting of Eudragit L, Eudragit S and Eudragit FS.

6. The capsule according to claim 4, wherein the modified-release coating further comprises at least one of a plasticizer, an anti-adhesive agent, a colorant and a binder.

7. The capsule according to any one of claims 1-6, wherein the modified-release coating comprises 50-80% of the film-forming agent (1), 5-15% of the plasticizer, 5-35% of the anti-adhesive agent, 0-15% of the binder and 0-8% of the colorant based on the total weight of the modified-release coating.

8. The capsule according to any one of claims 1-7, wherein the capsule is selected from the group consisting of a hydroxypropyl methylcellulose shell, a pullulan shell, a gelatin shell, a starch shell and a PVA-based shell.

9. The capsule according to any one of claims 1-8, wherein the isolating layer further comprises a film-forming agent (2).

10. The capsule according to claim 9, wherein the film-forming agent (2) is selected from the group consisting of hydroxypropyl methylcellulose, hydroxypropyl cellulose, methylcellulose, polyvinylpyrrolidone, polyvinyl alcohol and a mixture thereof.

11. The capsule according to any one of claims 1-10, wherein the isolating layer further comprises one or more of a plasticizer, an anti-adhesive agent and a colorant; the plasticizer is selected from one or more of triethyl citrate, tributyl citrate, dibutyl sebacate, dimethyl phthalate and polyethylene glycol; the anti-adhesive agent is selected from one or more of talc, silicon dioxide, magnesium stearate and glyceryl monostearate; the colorant is selected from titanium dioxide.

12. The capsule according to any one of claims 1-11, wherein a coating agent for the isolating layer is selected from Opadry II.

13. The capsule according to any one of claims 1-12, wherein the isolating layer comprises a mixture of Opadry II and at least one selected from the group consisting of sucrose, lactose, mannitol, starch and sorbitol.

14. The capsule according to claim 12 or 13, wherein a weight ratio of the hydrophilic molecule to the coating agent for the isolating layer is 1:15 to 10:1, preferably 1:10 to 1:5.

15. The capsule according to any one of claims 1-14, wherein the isolating layer is directly applied to the surface of the capsule in an amount of 5-50 mg/cm² based on the surface of the capsule.

16. The capsule according to any one of claims 1-15, wherein the modified-release coating comprises Eudragit L, Eudragit S, triethyl citrate and talc.

17. The capsule according to any one of claims 1-16, wherein the non-liquid filler comprises a nutritional ingredient and/or an active drug ingredient.

18. The capsule according to claim 17, wherein the drug is selected from at least one of a drug for treating intestinal diseases, a drug for treating glomerulonephritis, a drug for treating autoimmune hepatitis, a hypoglycemic drug, an antiviral drug, an anti-Parkinson drug and a nucleic acid-containing formulation for delivery to intestinal cells; the drug for treating intestinal diseases or glomerulonephritis or the drug for treating autoimmune hepatitis is preferably a corticosteroid, an anticholinergic drug and an opioid receptor antagonist; the hypoglycemic drug is preferably insulin and an analog thereof; the antiviral drug is preferably an anti-HIV drug.

19. The capsule according to claim 18, wherein the drug is a corticosteroid, preferably budesonide.

20. The capsule according to claim 19, wherein the capsule is filled with a budesonide-containing pharmaceutical composition, and the pharmaceutical composition comprises sustained-release components of a drug-containing core, an isolating layer and a sustained-release coating layer (1), wherein the drug-containing core is covered in the isolating layer and the isolating layer is covered in the sustained-release coating layer (1), wherein the drug-containing core comprises a corticosteroid and hydroxypropyl methylcellulose, and a weight ratio of hydroxypropyl methylcellulose to the corticosteroid is at least 2.5:1.

21. The capsule according to claim 19, wherein the capsule is filled with a levodopa-containing pharmaceutical composition, and the pharmaceutical composition comprises a levodopa-containing core and a sustained-release coating layer (1), wherein the levodopa-containing core is covered in the sustained-release coating layer.

22. A method for preparing the capsule according to any one of claims 1-20, comprising the following steps: filling the capsule body with a non-liquid filler, closing the capsule with the cap, applying the isolating layer onto the capsule shell, and applying the modified-release coating onto the isolating layer.

23. Use of the capsule according to any one of claims 1-22 in the preparation of a medicament for treating an inflammatory bowel disease, a glomerulonephritis disease or an autoimmune liver disease, wherein the inflammatory bowel disease is preferably Crohn's disease or ulcerative colitis, and the autoimmune liver disease is selected from autoimmune hepatitis.
